(19) Europäisches Patentamt
European Patent Office
Office européen des brevets

(11) Publication number: **0 016 650**
**A2**

# (12) EUROPEAN PATENT APPLICATION

(21) Application number: 80300894.5

(22) Date of filing: 21.03.80

(51) Int. Cl.³: C 07 C 49/417
C 07 C 45/62
//C07C45/74, C07C49/653,
A23L1/226, C11B9/00

(30) Priority: 26.03.79 JP 35337/79

(43) Date of publication of application:
01.10.80 Bulletin 80/20

(84) Designated Contracting States:
CH DE FR GB NL

(71) Applicant: NIPPON ZEON CO., LTD.
6-1, 2-chome
Marunouchi Chiyoda-ku, Tokyo(JP)

(72) Inventor: Shiozaki, Shozo
3-37-24, Mutsuura
Kanazawa-ku Yokohama-shi, Kanagawa-ken(JP)

(72) Inventor: Senuma, Mitsushi
1800-15, Kajiwara
Kamakura-shi Kanagawa-ken(JP)

(72) Inventor: Furumai, Shigehiro
873, Futoo-cho
Kohoku-ku Yokohama-shi, Kanagawa-ken(JP)

(72) Inventor: Kawashima, Horoshi
873, Futoo-cho
Kohoku-ku Yokohama-shi, Kanagawa-ken(JP)

(74) Representative: Myerscough, Philip Boyd et al,
J.A. KEMP & CO. 14, South Square
Gray's Inn London, W.C. 2(GB)

## (54) 2-Cyclopentyl-cyclopentanone, fragrance or flavouring compositions containing it and their use.

(57) 2-Cyclopentyl-cyclopentanone is a novel compound which has been found to be suitable for use as a perfuming or odor-modifying ingredient of fragrance or flavoring compositions, the compositions themselves being useful in the perfuming or flavoring of, for example, dentifrices etc. 2-Cyclopentyl-cyclopentanone has a fresh, cool perfume or flavor and can enhance the perfume or flavor of other perfuming or odor-modifying ingredients. It can readily be produced from cyclopentanone.

EP 0 016 650 A2

TITLE MODIFIED
see front page

0016650



DESCRIPTION

"2-CYCLOPENTYL-CYCLOPENTANONE, FRAGRANCE OR FLAVORING COMPOSITIONS CONTAINING IT, AND USE OF SAID COMPOSITIONS IN THE PERFUMING OR FLAVORING OF, FOR EXAMPLE, DENTIFRICES"

This invention relates to 2-cyclopentyl-cyclopentanone, fragrance or flavoring compositions containing 2-cyclopentyl-cyclopentanone as perfuming or odor-modifying ingredients, and the use of such compositions.

In recent years, ingredients that give various perfuming or flavoring notes, particularly fresh, clean, cool and green notes, have been incorporated in fragrance or flavoring compositions for use in dentifrices, mouth fresheners, chewing gums, foodstuffs, beverages, shampoos, toilet soaps, cosmetics, household materials, detergents, etc. It is common practice to prepare such fragrance or flavoring compositions by suitably combining various known perfuming or odor-modifying ingredients or incorporating them in varying proportions. However, combinations of known perfuming or odor-modifying ingredients are not entirely satisfactory, and the development of novel perfuming or odor-modifying ingredients affording fresh, clean and cool notes has been highly desired.

The invention provides fragrance or flavoring compositions having enhanced and/or different odors or flavors than the conventional ones, by using a novel perfuming or odor-modifying ingredient that can impart fresh and clean and/or cool notes to fragrance or flavoring compositions.

The present invention is based upon the finding that a novel substance, 2-cyclopentyl-cyclopentanone, exhibits an excellent perfuming or odor-modifying effect.

Thus, the present invention provides a fragrance or flavoring composition containing as one of its active ingredients a novel substance, 2-cyclopentyl-cyclopentanone, expressed by the following formula:

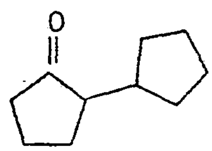

2-Cyclopentyl-cyclopentanone can readily be synthesized by known synthesizing methods, for example, the methods described in Roland Mayer, Chem. Ber. 89, 1443 (1956); Martin Cordon, et al., J. Am. Chem. Soc., 76, 1643 (1954); and Georges Le Guillanton, Bull. Soc. Chim. France, 1963(3), 611. A preferred method in terms of yield, operability, and availability of starting material involves condensing cyclopentanone in the presence of a basic condensation agent and hydrogenating the resulting 2-cyclopentylidene-cyclopentanone.

2-Cyclopentyl-cyclopentanone possesses light green notes which are fresh and cool and minty notes which are strong, sharp and cool. Therefore, 2-cyclopentyl-cyclopentanone imparts fresh, clean and/or cool notes to various fragrance or flavoring compositions when incorporated in them. The fragrance or flavoring compositions can be used in order to modify or improve the olfactory properties of materials such as dentifrices, mouth fresheners, chewing gums, foodstuffs, beverages, shampoos, soaps, cosmetics, household materials, detergents, cleaning products, paints, papers, etc. The amount of 2-cyclopentyl-cyclopentanone added to a fragrance or flavoring composition can vary according to the type of the the fragrance or flavoring composition, the specific organoleptic effects required, and so forth. Usually, that amount is 0.001 to 40% by weight, preferably 0.01 to 15% by weight, based on the fragrance or flavoring

composition.

2-Cyclopentyl-cyclopentanone is stable and can be easily mixed with other perfuming or odor-modifying ingredients. It is also economical because it is produced from an ordinary starting material with ease and at low cost.

The present invention will be described in greater detail with reference to the following examples.

Referential Example

A 5-liter three-necked flask equipped with a stirrer and a reflux condenser was charged with 840 g of cyclopentanone and 840 g of a 10% aqueous solution of sodium hydroxide, and they were reacted at 90°C. for 3 hours. Then, the mixed solution was cooled to room temperature, and the aqueous phase was removed by a separating funnel. The organic phase was washed with 840 g of water, 840 g of a 5% aqueous solution of hydrogen chloride, 840 g of a 5% aqueous solution of $NaHCO_3$, and 840 g of water in this order, then dried over anhydrous sodium sulfate, and distilled at a reduced pressure of 1.5 mmHg, to obtain 405 g of a fraction distilling off at 71.5°C. Its infrared spectrum, NMR and mass spectrum confirmed that the fraction was 2-cyclopentylidene-cyclopentanone.

To 380 g of the 2-cyclopentylidene-cyclopentanone was added 17.0 g of 5% palladium carried on activated carbon as catalyst, and the 2-cyclopentylidene-cyclopentanone was hydrogenated at ordinary temperature and ordinary pressure. After completion of the hydrogenation, the catalyst was removed by filtration, and the residue was subjected to precision distillation at a reduced pressure of 3.5 mmHg, thereby to afford 342 g of a fraction distilling out at 79.5°C. Its infrared spectrum, NMR and mass spectrum confirmed that the fraction was 2-cyclopentyl-cyclopentanone. Gas chromatography also confirmed that its purity was 99.8%. The identifying data of this compound were as follows:

IR: 1730 $cm^{-1}$ (>C=O)

$^{13}$C-NMR: 219.72 (C=O, S); 52.73 (CH, d); 40.02 (CH, d); 38.50 ($CH_2$, t); 30.76 ($CH_2$, t); 29.65 ($CH_2$, t); 27.48 ($CH_2$, t); 25.14 ($CH_2$, t); 24.83 ($CH_2$, t); 20.62 ($CH_2$, t) $\delta$ ppm (from TMS)

MS (m/e): 152 (15); 123 (10); 109 (10); 85 (30); 84 (100); 83 (30); 67 (50); 55 (25); 54 (23); 53 (20); 41 (27); 39 (23)

Example 1

The following materials were mixed to prepare a fragrance composition for use in a dentifrice.

| | parts by weight |
|---|---|
| Peppermint oil | 50 |
| Anethole | 23 |
| Clove oil | 10 |
| Cinnamon oil | 7 |
| Benzoin | 4.5 |
| Rose oil | 0.5 |
| 2-Cyclopentyl-cyclopentanone | 10 |

The resulting fragrance composition had perfuming notes with higher coolness and much more freshness than the corresponding composition free from 2-cyclopentyl-cyclopentanone.

Example 2

The following substances were mixed to prepare a fragrance composition for use in a soap.

| | Parts by weight |
|---|---|
| Heliotropin | 50 |
| Phenylethyl alcohol | 8 |
| Terpineol | 7.5 |
| Benzyl acetate | 6 |
| Linalol | 5.5 |
| Coumarin | 5 |
| Cananga oil | 5 |
| Linalyl acetate | 4.5 |
| Sandalwood oil | 3.5 |
| Styrax | 3 |
| Musk ambrette | 2 |
| 2-Cyclopentyl-cyclopentanone | 2 |

The resulting fragrance composition had perfuming notes much more accentual and nobler than the corresponding composition not containing 2-cyclopentyl-cyclopentanone.

Example 3

The substances indicated below were mixed to obtain a fragrance or flavoring composition having a jasmine-like smell.

| | Parts by weight |
|---|---|
| Hydroxycitronellal | 25 |
| Benzyl acetate | 20 |
| Linalol | 10 |
| Phenylethyl alcohol | 10 |
| Phenylethyl isobutyrate | 9 |
| α-Ionone | 5 |
| Benzyl salicylate | 5 |
| Amylcinnamaldehyde | 3 |
| P-Cresyl phenylacetate | 3 |
| Jasmine absolute | 3 |
| Musk ketone | 2 |
| Jasmone | 1 |
| Ylang-ylang oil | 1 |
| Phenylacetaldehyde | 1 |
| Civet | 0.5 |
| $C_{10}$ aldehyde | 0.3 |
| Indole | 0.1 |
| Octyl isobutyrate | 0.1 |
| 2-Cyclopentyl-cyclopentanone | 2 |

The fragrance or flavoring composition had a by far stronger jasmine-like smell than the corresponding composition free from 2-cyclopentyl-cyclopentanone.

## CLAIMS

1. 2-Cyclopentyl-cyclopentanone.

2. A fragrance or flavoring composition comprising at least one perfuming or odor-modifying ingredient characterised by containing 2-cyclopentyl-cyclopentanone as an active ingredient.

3. A fragrance or flavoring composition according to claim 2 characterised by containing 0.001 to 40% by weight of the 2-cyclopentyl-cyclopentanone.

4. A fragrance or flavoring composition according to claim 3 characterised by containing 0.01 to 15% by weight of the 2-cyclopentyl-cyclopentanone.

5. The use of a fragrance or flavoring compositions as claimed in claim 2, 3 or 4 in the perfuming or flavoring of dentifrices, mouth fresheners, chewing gums, foodstuffs, beverages, shampoos, soaps, cosmetics, household materials or detergents.